# EUROPEAN PATENT SPECIFICATION

(11) **EP 2 003 130 B1**
(45) Date of publication and mention of the grant of the patent: **16.11.2011**
(21) Application number: 07715278.3
(22) Date of filing: 07.03.2007
(51) Int. Cl.: C07D 401/12, A61K 31/4439, A61K 45/00, A61K 47/36, A61P 1/04, A61P 1/18, A61P 31/04, A61P 35/00, A61P 43/00

(54) **NOVEL PYRIDINE DERIVATIVE HAVING ANTI-HELICOBACTER PYLORI ACTIVITY**
NEUES PYRIDINDERIVAT MIT WIRKUNG GEGEN HELICOBACTER PYLORI
NOUVEAU DERIVE DE PYRIDINE AYANT UNE ACTIVITE CONTRE HELICOBACTER PYLORI

(30) Priority: 10.03.2006 JP 2006066431
(43) Date of publication of application: 17.12.2008
(73) Proprietor: aRigen Pharmaceuticals, Inc., Minato-ku Tokyo 107-0062 (JP)
(72) Inventor: ITO, Masaharu, Minato-ku, Tokyo 107-0062 (JP); YAMAMOTO, Masaichi, Minato-ku, Tokyo 107-0062 (JP)
(74) Representative: Benson, John Everett
(86) International application number: PCT/JP2007/054387
(87) International publication number: WO 2007/105551

(56) References cited:
- EP-A1- 0 175 464
- WO-A1-97/23207
- JP-A- 01 006 270
- JP-A- 03 173 817
- JP-A- 07 126 189
- JP-A- 61 050 978
- US-A1- 2003 036 656
- THOMAS C. KÜHLER, MARIANNE SWANSON, VLADIMIR SHCHERBUCHIN ET AL.: "Structure-activity relationship of 2-[[(2-pyridyl)methyl]thio]-1H-benzimidazo les as anti Heliobacter pylori agents in vitro and evaluation of their in vivo efficacy", J. MED. CHEM., vol. 41, 1999, pages 1777-1788, XP002609000,

## Description

### Technical Field

The present invention relates to a novel pyridine derivative having an excellent anti-*Helicobacterpylori* action, a method for producing the compound, and a pharmaceutical composition comprising the compound.

### Background Art

Gastritis, gastric ulcer and duodenal ulcer are diseases caused by a complicated combination of factors such as stress, genetic predisposition and lifestyle habits. In recent years, as one cause of the diseases, *Helicobacter pylori* (*H. pylori*) have been brought to spotlight. Since Warren and Marshall succeeded in the isolation and culture of a helical-shaped bacterium from stomach biopsy samples in 1983, intensive research has been carried out on the relationship between gastritis, gastric ulcer, duodenal ulcer and gastric cancer, and the subj ect bacterium. As a result, the infection rate of *H. pylori* was such that the positive rate is about 4% in normal stomachs, whereas the positive rate is as high as about 83% in chronic gastritis, about 69% in gastric ulcer, about 92% in duodenal ulcer, and about 51% in non-ulcer dyspepsia syndrome (Martin J. Blaser,: Clin. Infectious Disease, 15; 386-393, 1992). Furthermore, *H. pylori* infection is strongly relevant to the incidence rate of gastric cancer, and thus the International Agency for Research on Cancer affiliated with the World Health Organization (WHO) decided in 1994 that *H. pylori* is a strongly causative oncogenic factor.

For the treatment for gastritis, gastric ulcer, duodenal ulcer and the like, symptomatic therapies in which H2 blockers suppressing the secretion of gastric acid, drugs suppressing the secretion of gastric acid, such as proton pump inhibitors, and mucous protective drugs and the like are used, constitute the mainstream of the treatment. However, it is said that although these drugs temporarily cure lesions, when the treatment is ceased, recurrence occurs in about 80% of the cases within one year (Martin J. Blaser,: Clin. Infectious Disease, 15; 386-393, 1992). On the other hand, it is reported that when *Helicobacter pylori* (*H. pylori*) was eradicated, the recurrence rate in one year was within 10% for duodenal ulcer, and also an obviously low rate for gastric ulcer (Graham D.Y., et al.: Ann. Intern. Med., 116;705-708, 1992). There, methods of simultaneously administering antibacterial agents such as amoxicillin or clarithromycin and metronidazole to the proton pump inhibitors (PPI), over one week or more in large quantities, have become popular. However, administration of antibacterial agents in large quantities kills useful bacteria in the intestinal tract as well. As a result, it is apprehended that there is a possibility of promoting soft feces, diarrhea and dysgeusia; side effects such as glossitis, stomatitis, hepatic malfunction, hepatic dysfunction and hemorrhagic enteritis, and the appearance of methicillin resistant *Staphylococcus aureus* (MRSA).

Under such circumstances, there have been attempts to develop a highly safe medicament exhibiting a sufficient antibacterial action against *H. pylori* with conventionally used doses. For example, Patent Documents 1 to 4 and 9 propose such medicaments.

In clinical practice, in order for a substance to exhibit an effect of eradicating *H. pylori* which is equivalent to the effects of antibiotic substances, it is necessary for the substance to show an activity equivalent to or better than the anti-*H. pylori* activity of those antibiotic substances showing clinical effectiveness against *H. pylori.* That is, it is desirable that the activity is stronger than an activity as represented by a minimum inhibitory concentration (MIC) of 0.3 µg/ml.

Furthermore, some compounds of the guanidinomethylcyclo hexanecarboxylic acid ester derivatives described in Patent Document 5 exhibit anti-*H. pylori* activities as represented by an MIC of less than 1 µg/ml. However, these compounds have a property of very rapidly being decomposed by degrading enzymes in the small intestine or in blood. This property is associated with the compounds which have been designed to have selectivity to *H. pylori* in accordance with the metabolic property of being degraded in the intestine or in blood, on the basis of the idea that "an antibiotic substance or a synthetic antibacterial agent is metabolically distributed when administered, such as that the substance is absorbed from the intestinal tract through the digestive tract to be incorporated into the blood, or is excreted along with feces, and thus many bacteria that inhabit in the intestine happen to be annihilated by the drug passing through the intestinal tract, thereby breaking the balance of the intestinal bacterial flora. Thus, administration over a long time period must be avoided," as described in Patent Document 6. However, it is known that the metabolic enzymes in the intestine or in the blood and the intestinal bacteria have individual variation or fluctuations derived from diet. Therefore, it is not highly probable that such metabolic characteristics are stabilized and assured in patients having various patient backgrounds.

Meanwhile, there are known pyridine derivatives which are useful as anti-ulcerative agents (see Patent Document 7), pyridine derivatives exhibiting an antibacterial action against *Helicobacter pylori* (see Patent Document 2), and pyridine derivatives used in suppressing the secretion of gastric acid (see Patent Document 8). In addition, the compounds of the Comparative Examples that will be described later, that is, 2-[{4-(2-hydroxyethoxy)-3-methylpyridin-2-yl}methylthio]-1H -benzimidazole (Comparative Compound 1) and 2-[{4-(3-hydroxy propoxy)-3-methylpyridin-2-yl}methylthio]-1H-benzimidazole (Comparative Compound 2), are compounds that are described to be useful as anti-ulcerative agents in Example 26 and Example 34 in Patent Document 9. However, no description is provided on the experimental data related to the anti-ulcerative action, and there is no description or suggestion on the action against *Helicobacter pylori.*
Patent Document 1: JP-A No. 2-209809
Patent Document 2: JP-A No. 3-173817
Patent Document 3: JP-A No. 3-48680
Patent Document 4: JP-A No. 7-69888
Patent Document 5: WO 96/06825
Patent Document 6: WO 97/23207
Patent Document 7: JP-A No. 61-50979
Patent Document 8: JP-A No. 58-39622
Patent Document 9: JP-A No. 5-247035
US 2003/0036656 provides a method for obtaining derivatives of the formula 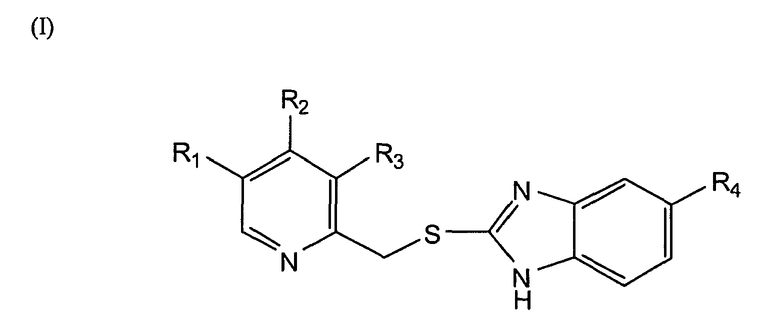 where each of R₁, R₃ and R₄, independently of each other, is hydrogen, an alkyl, alkoxy or fluorinated alkoxy of 1 to 6 carbon atoms, and R₂ is nitro, halogen, alkoxy or halogenated alkoxy of 1 to 6 carbon atoms, or a group -OR-(CH₂)ₙ-OR₈, where n is an integer between 1 and 6 and R₈ represents hydrogen or an alkyl group with 1 to 6 carbon atoms. The compounds are useful as intermediates in the preparation of [[(pyridyl-substituted)methyl]sulfinyl]benzomidazol derivatives.

### Disclosure of the Invention

### Problems to be Solved by the Invention

Under such circumstances, the inventors of the present invention succeeded in the search for a compound which has a strong anti-*H.pylori* activity as represented by an MIC of less than 0.3 µg/ml, exerts no effect on the resident bacteria of human being, and specifically exhibits an antibacterial action against *H. pylori,* and also discovered a substance exhibiting effectiveness even against those bacteria which are resistant to antibiotic substances such as roxithromycin and ofloxacin, thus completing the present invention.

Thus, it is an object of the present invention to provide a compound exhibiting an excellent antibacterial action against *H. pylori,* and a pharmaceutical composition comprising the compound.

### Means for Solving the Problems

The present invention provides the following inventions of (1) to (12).

(1) A novel pyridine derivative represented by formula (I):

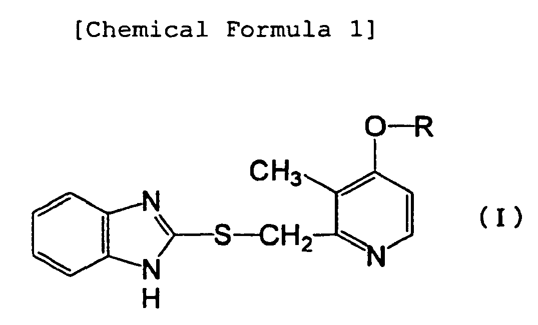

wherein R represents a straight-chained or branched hydroxyalkyl group having 5 to 10 carbon atoms, or a pharmaceutically acceptable salt thereof.

(2) A method for producing a novel pyridine derivative represented by formula (I):

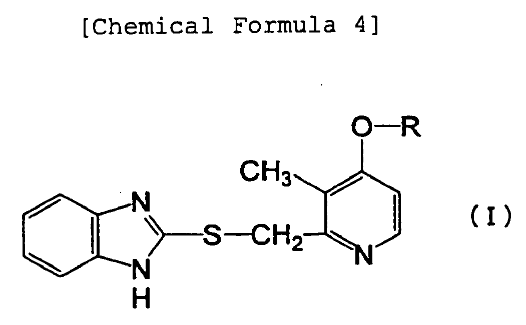

wherein R represents a straight-chained or branched hydroxyalkyl group having 5 to 10 carbon atoms, or a pharmaceutically acceptable salt thereof, the method comprising reacting a compound represented by formula (II):

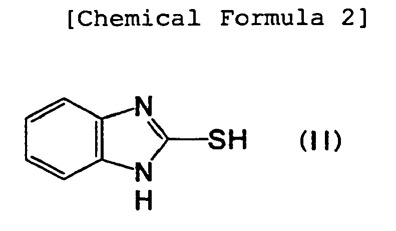

with a compound represented by formula (III):

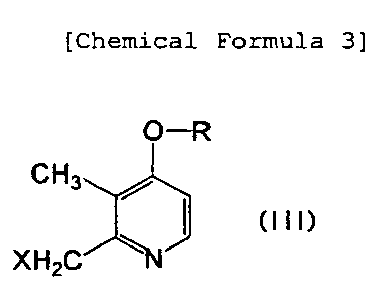

wherein R has the same meaning as defined above; and X represents a halogen atom or a sulfonyloxy group.

(3) A pharmaceutical composition comprising the novel pyridine derivative according to (1) or a pharmaceutically acceptable salt thereof.

(4) An anti-*Helicobacterpylori* agent comprising the novel pyridine derivative according to (1) or a pharmaceutically acceptable salt thereof.

(5) The anti-*Helicobacter pylori* agent according to (4), wherein the *Helicobacter pylori* to be treated is a bacterium resistant to macrolide-based antibiotic substances or new quinolone-based antibiotic substances.

(6) The anti-*Helicobacter pylori* agent according to (4), further comprising one or two or more dextrins.

(7) The anti-*Helicobacter pylori* agent according to (4), further comprising one or two or more drugs suppressing the secretion of gastric acid.

(8) A prophylactic or therapeutic agent for a disease associated with *Helicobacter pylori,* the agent comprising the novel pyridine derivative according to (1) or a pharmaceutically acceptable salt, as an active ingredient.

(9) The prophylactic or therapeutic agent according to (8), wherein the disease is gastritis, gastric ulcer, duodenal ulcer, non-ulcer dyspepsia syndrome, gastric MALT lymphoma, hyperplastic polyp of the stomach, gastric cancer, digestive system cancer, pancreatitis or inflammatory bowel disease.

(10) The prophylactic or therapeutic agent according to (9), wherein the gastric cancer is a gastric cancer developing after endoscopic excision of early gastric cancer.

(11) The prophylactic or therapeutic agent according to any one of (8) to (10), further comprising one or two or more dextrins.

(12) The prophylactic or therapeutic agent according to any one of (8) to (10), further comprising one or two or more drugs suppressing the secretion of gastric acid.

(13) A method for preventing or treating a disease in a mammal, the method comprising administering the novel pyridine derivative according to (1) or a pharmaceutically acceptable salt to the mammal.

(14) The method according to (13), further comprising administering one or two or more dextrins, or one or two or more drugs suppressing the secretion of gastric acid.

(15) A method for eradicating or controlling *Helicobacter pylori* in a mammal, the method comprising administering to the mammal in need thereof, the novel pyridine derivative according to (1) or a pharmaceutically acceptable salt thereof in an amount effective for eradicating or controlling *Helicobacter pylori.*

(16) The method according to (15), further comprising one or two or more dextrins, or one or two or more drugs suppressing the secretion of gastric acid.

(17) A method for preventing or treating a disease associated with *Helicobacter pylori* in a mammal, the method comprising administering to the mammal in need thereof, the novel pyridine derivative according to (1) or a pharmaceutically acceptable salt thereof in an amount effective for preventing or treating the disease associated with *Helicobacter pylori.*

(18) The method according to (17), further comprising administering one or two or more dextrins, or one or two or more drugs suppressing the secretion of gastric acid.

(19) Use of the novel pyridine derivative according to (1) or a pharmaceutically acceptable salt thereof, for the manufacture of a medicine.

(20) The use according to (19), wherein the novel pyridine derivative according to (1) or a pharmaceutically acceptable salt thereof is used in combination with one or two or more dextrins, or with one or two or more drugs suppressing the secretion of gastric acid.

(21) The use according to (19) or (20), wherein the medicine is a medicine for preventing or treating a disease associated with *Helicobactor pylori*.

(22) Use of the novel pyridine derivative according to (1) or a pharmaceutically acceptable salt thereof, for the manufacture of an anti*-Helicobacter pylori* agent.

(23) The use according to (22), wherein the novel pyridine derivative according to (1) or a pharmaceutically acceptable salt thereof is used in combination with one or two or more dextrins, or with one or two or more drugs suppressing the secretion of gastric acid.

(24) A product comprising the novel pyridine derivative according to (1) or a pharmaceutically acceptable salt thereof, and an instruction or packaging container describing that the compound is used for eradicating or controlling *Helicobacter pylori*.

(25) A product comprising the novel pyridine derivative according to (1) or a pharmaceutically acceptable salt thereof, and an instruction or packaging container describing that the compound is used for preventing or treating a disease associated with *Helicobacter pylori.*

### Effects of the Invention

The novel pyridine derivative of the present invention and pharmaceutically acceptable salts thereof exhibit an excellent antibacterial action against *Helicobacter pylori* (*H. pylori*). The novel pyridine derivative of the present invention and pharmaceutically acceptable salts thereof have a very excellent advantage as a medicine that the compounds exert no effect on resident bacteria of human being but specifically exhibit an antibacterial action against *H. pylori,* and also have an advantage that the compounds exhibit an excellent antibacterial action against *H. pylori* which is resistant to macrolide-based antibiotic substances or new quinolone-based antibacterial agents. Furthermore, use of the novel pyridine derivative of the present invention and pharmaceutically acceptable salts thereof allows eradication of *H. pylori* in a mammal (particularly, human being).

The present specification includes the subject matter described in the specification and/or drawings of Japanese Patent Application No. 2006-66431, which is the foundation of the priority of the present patent application.

### Best Mode for Carrying Out the Invention

R in the formula (I) or (III) represents a straight-chained or branched hydroxyalkyl group having 5 to 10 carbon atoms. The "straight-chained or branched hydroxyalkyl group having 5 to 10 carbon atoms" according to the present invention is a group in which at least one hydrogen group of a straight-chained or branched alkyl group having 5 to 10 carbon atoms is substituted by a hydroxyl group. The number of hydroxyl groups on the hydroxyalkyl group is not particularly limited, but is preferably 1 to 3, and more preferably 1 to 2, and most preferably one. The position of the hydroxyl group in the hydroxyalkyl group is not particularly limited, and the hydroxyl group may be present at any position on the carbon chain. However, it is preferable that one hydroxyl group (if a plurality of hydroxyl groups are present, at least one among those) is present on a carbon atom at one end of the carbon chain. That is, at least one hydroxyl group on the hydroxyalkyl group is preferably a group formed by substituting a hydrogen group on -CH₃ at one end of a straight-chained or branched alkyl group having 5 to 10 carbon atoms. The carbon number of the R group would be suitably any number within the range of 5 to 10, but it is particularly preferable that the carbon number is 8. The carbon number of the R group is preferably in the range of 5 to 10, more preferably in the range of 6 to 9, and particularly preferably 8, from the viewpoint of the intensity of the anti-*Helicobacter pylori* activity. The R group may be any of a straight chain and a branched chain, but it is particularly preferable that the group is straight-chained. Particularly preferred examples of the R group include -(CH₂)₅OH, -(CH₂)₆OH, -(CH₂)₇OH, -(CH₂)₈OH, -(CH₂)₉OH, and -(CH₂)₁₀OH.

In the formula (III), X represents a halogen atom or a sulfonyloxy group. The halogen atom means any of fluorine, chlorine, bromine and iodine. As the sulfonyloxy group, various sulfonyloxy groups can be used, andtypicallyanalkylsulfonyloxy group which may be substituted, or an arylsulfonyloxy group which may be substituted can be used. Specifically, the alkylsulfonyloxy group may be exemplified by a lower alkylsulfonyloxy group such as a methanesulfonyloxy group or an ethanesulfonyloxy group. The alkyl contained in the alkylsulfonyloxy group may be further substituted with a substituent such as halogen. The arylsulfonyloxy group may be exemplified by a benzenesulfonyloxy group or the like. The aryl contained in the arylsulfonyloxy group may be further substituted with a substituent.

The pyridine derivative (I), which is the target compound of the present invention, can be produced by allowing raw material compounds (II) and (III) to react. It is favorable that the subj ect reaction is performed in the presence of a base. Examples of the base include alkali metal hydrides such as sodium hydride and potassium hydride; alcoholates such as potassium t-butoxide, sodium propoxide, sodium ethoxide and sodium methoxide; alkali metal carbonates such as potassium carbonate and sodium carbonate; organic amines such as triethylamine; and the like. As the solvent to be used in the reaction, for example, alcohols such as methanol and ethanol, dimethylsulfoxide and the like may be mentioned. The amount of the base used in the reaction is usually a slightly excess amount with respect to one equivalent, but a large excess of base may also be used. That is, the amount is 1 to 10 equivalents, and preferably 1 to 4 equivalents. The reaction temperature is typically from -40°C to a temperature near the boiling point of the solvent used, and preferably 0°C to 60°C. The reaction time is about 0.2 to 24 hours, and preferably 0.5 to 2 hours.

The target compound (I) produced by the reaction can be isolated and purified by conventionally used means such as recrystallization and chromatography.

The compound (I) of the present invention may be converted to a pharmacologically acceptable salt by conventionally used means. Examples of such salt include hydrochloride, hydrobromide, hydroiodide, phosphate, nitrate, sulfate, acetate, citrate and the like.

The compound according to the present invention or a salt thereof may be in the form of hydrate, or a solvate with a lower alcohol or the like. The scope of the compound according to the present invention or a salt thereof includes those hydrate or solvate forms as well.

A method for producing the raw material compound (III) will be described in the following.

### Production Method 1

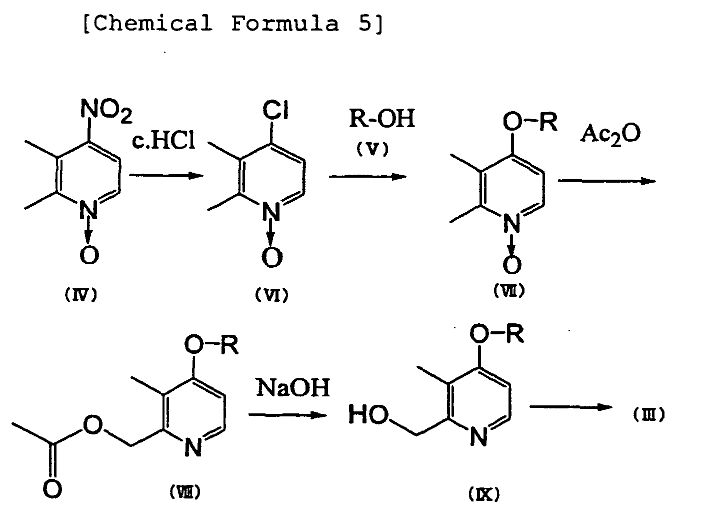

A chloro derivative (VI) can be obtained by reacting a nitro compound represented by formula (IV) with concentrated hydrochloric acid. When the chloro derivative (VI) is reacted with an alcohol derivative ROH (IV) in the presence of a base, an alkoxy derivative of formula (VII) can be obtained. The base includes, for example, alkali metals such as lithium, sodium and potassium; alkali metal hydrides such as sodium hydride and potassium hydride; alcoholates such as potassium t-butoxide, sodium propoxide, sodium ethoxide and sodium methoxide; carbonates or hydrogen carbonates of alkali metals such as potassium carbonate, lithium carbonate, sodium carbonate, potassium hydrogen carbonate and sodium hydrogen carbonate; alkali metals such as potassium, sodium and lithium; alkaline hydroxides such as sodium hydroxide and potassium hydroxide; and the like. As the solvent to be used in the reaction, in addition to lower alcohols represented by ROH, ethers such as tetrahydrofuran, dioxane and t-butyl methyl ether; ketones such as acetone and methyl ethyl ketone; and aromatic hydrocarbons such as benzene, toluene, xylene and trimethylbenzene, may be mentioned. Examples of other usable solvents include acetonitrile, dimethylformamide, dimethylsulfoxide, N-methylpyrrolidone and the like. As for the reaction temperature, a suitable temperature is selected from -70°C to a temperature near the boiling point of the solvent. The reaction time is about 1 to 48 hours.

When the compound (VII) thus obtained is heated (about 80 to 120°C) in the presence of acetic anhydride alone or an acetate of an alkali metal, such as sodium acetate or potassium acetate, or in the presence of a mineral acid such as sulfuric acid or perchloric acid, a 2-acetoxymethylpyridine derivative represented by formula (VIII) is obtained. The reaction time is typically about 0.1 to 10 hours.

Subsequently, a 2-hydroxymethylpyridine derivative represented by formula (IX) can be produced by subjecting the compound (VIII) to alkaline hydrolysis. Examples of the alkali include sodium hydroxide, potassium hydroxide, potassium carbonate, sodium carbonate and the like. As the solvent to be used, for example, methanol, ethanol, water and the like may be mentioned. The reaction time is about 0.1 to 2 hours.

Then, a 2-halogenomethylpyridine derivative represented by formula (III) can be produced by halogenating the compound (IX) with a chlorinating agent such as thionyl chloride, a brominating agent or an iodinating agent. Alternatively, a 2-sulfonyloxymethylpyridine derivative represented by formula (III) can be produced by performing sulfonyloxylation with various sulfonyloxylating agents, for example, an alkylsulfonyloxylating agent such as methanesulfonyl chloride or ethanesulfonyl chloride, or an aromatic sulfonyloxylating agent such as benzenesulfonyl chloride. As the solvent to be used, for example, chloroform, dichloromethane, tetrachloroethane, benzene, toluene, tetrahydrofuran, dioxane, methyl t-butyl ether, dioxane, dimethylformamide, dimethylsulfoxide, N-methylpyrrolidone and the like may be mentioned. As for the reaction temperature, a suitable temperature is selected typically from -70°C to a temperature near the boiling point of the solvent. The reaction time is about 0.1 to 2 hours.

The compound of the present invention or a salt thereof can eradicate or control *Helicobacter pylori* in the body of an animal belonging to mammal (typically, human being). That is, the compound of the present invention or a salt thereof is effective as an anti-*Helicobacter pylori* agent.

The present invention also provides a method for eradicating or controlling *Helicobacter pylori* in a mammal, the method comprising administering an effective amount of the compound of the present invention or a salt thereof to a mammal in need of the method.

The present invention also provides a use of the compound of the present invention or a salt thereof, for the manufacture of an anti-*Helicobacter pylori* agent.

A medicament containing the compound of the present invention or a salt thereof is effective for preventing or treating a disease associated with *Helicobacter pylori.* The "disease associated with *Helicobacter pylori*" according to the present invention refers to a disease which is caused or exacerbated by the infection, survival or propagation of *Helicobacter pylori* in the living body. In other words, the "disease associated with *Helicobacter pylori*" is a disease in which the symptoms may be improved by removing *Helicobacter pylori*. Examples of such disease include gastritis, gastric ulcer, duodenal ulcer, non-ulcer dyspepsia syndrome, gastric MALT lymphoma, hyperplastic polyp of the stomach, gastric cancer (particularly, gastric cancer developing after endoscopic excision of early gastric cancer) and the like. Other examples of the "disease associated with *Helicobacter pylori*" include digestive system cancer and pancreatitis caused by *Helicobacter pylori.* The compound of the present invention or a salt thereof can delay or impede the progress of digestive system cancer caused by *Helicobacter pylori.* As another example of the "disease associated with *Helicobacter pylori*," inflammatory bowel disease caused by *Helicobacter pylori* may be mentioned.

Upon using the compound of the present invention as a medicine, various dosage forms can be employed according to the purpose of prevention or treatment, and examples of the administrable formation include powders, fine granules, granules, tablets, capsules, dry syrups, syrups, injections and the like. A pharmaceutical composition containing the compound of the present invention can include a pharmaceutically acceptable carrier or excipient, or other additives.

In the case of preparing an oral solid preparation, an excipient, and if necessary, a binding agent, a disintegrant, a gliding agent, a colorant, a savoring or flavoring agent and the like can be added to the compound of the present invention, and then tablets, coated tablets, granules, powders, capsules and the like can be produced by conventional methods. As for such additives, those generally used in the related art may be used. For example, as the excipient, corn starch, lactose, sucrose, sodium chloride, mannitol, sorbite, glucose, starch, calcium carbonate, kaolin, microcrystalline cellulose, silicic acid and the like can be used. As the binding agent, water, ethanol, gum arabic, tragacanth, propanol, simple syrup, glucose solution, starch solution, gelatin solution, carboxymethylcellulose, hydroxypropylcellulose, gelatin, hydroxypropyl starch, methylcellulose, ethylcellulose, shellac, calcium phosphate, polyvinyl alcohol, polyvinyl ether, polyvinylpyrrolidone and the like can be used. As the disintegrant, powdered gelatin, crystalline cellulose, dry starch, sodium alginate, pectin, powdered agar, carboxymethylcellulose, sodium hydrogen carbonate, calcium carbonate, calcium citrate, sodium lauryl sulfate, stearic acid monoglyceride, lactose and the like can be used. As the gliding agent, silica, purified talc, stearic acid salts, borax, polyethylene glycol and the like can be used. As the colorant, those having been approved for addition, such as titanium oxide and iron oxide, can be used. As the savoring or flavoring agent, sucrose, orange peel, citric acid, tartaric acid and the like can be used.

In the case of preparing an oral liquid preparation, a savoring agent, a buffering agent, a stabilizer, a flavoring agent and the like can be added to the compound of the present invention, and then liquid preparations for internal use, syrups, elixirs and the like can be produced by conventional methods. In this case, as the savoring or flavoring agent, those mentioned above may be used. As the buffering agent, sodium citrate and the like may be mentioned. As the stabilizer, tragacanth, gum arabic, gelatin and the like may be mentioned.

In the case of preparing an injectable preparation, a pH adjusting agent, a buffering agent, a stabilizer, an isotonic agent, a local anesthetic and the like can be added to the compound of the present invention, and then injectable preparations for subcutaneous, intramuscular and intravenous uses can be produced by conventional methods. As the pH adjusting agent and buffering agent in this case, sodium citrate, sodium acetate, sodium phosphate and the like may be mentioned. As the stabilizer, sodium pyrosulfite, EDTA, thioglycolic acid, thiolactic acid and the like may be mentioned. As the local anesthetic, procaine hydrochloride, lidocaine hydrochloride and the like may be mentioned. As the isotonic agent, sodium chloride, glucose and the like may be mentioned as examples.

The pharmaceutical composition and prophylactic or therapeutic agent of the present invention described in the claims can further contain one or two or more dextrins, in addition to the novel pyridine derivative represented by the formula (I) or a pharmaceutically acceptable salt thereof. Examples of the dextrin that can be used in the present invention include α-dextrin, β-dextrin, γ-dextrin, α-cyclodextrin, β-cyclodextrin, γ-cyclodextrin and the like, but are not limited to these.

The pharmaceutical composition and prophylactic or therapeutic agent of the present invention described in the claims can further contain one or two or more drugs suppressing the secretion of gastric acid, in addition to the novel pyridine derivative represented by the formula (I) or a pharmaceutically acceptable salt thereof. As the drug suppressing the secretion of gastric acid, H2 blockers, proton pump inhibitors and the like may be mentioned. Examples of the H2 blocker that can be used in the present invention include famotidine, ranitidine and the like, while examples of the proton pump inhibitor that can be used in the present invention include lansoprazole, omeprazole, rabeprazole, pantoprazole and the like, but the examples are not to be limited to these.

By the use of a combined preparation as described above, the effects of the present inventions are expected to be further enhanced.

The amount of the compound of the present invention to be incorporated in each of the unit dosage forms may vary depending on the symptoms of the patient in need of application of the compound, or on the formulation, but in general, it is desirable to set the amount in each unit dosage form to about 1 to 1200 mg for oral preparations, and about 0.1 to 500 mg for injectable preparations. Furthermore, the amount of daily administration of a medicament being in the above-described dosage form, varies with the symptoms, body weight, age, gender and the like of the patient, and thus cannot be determined as a fixed value; however, the amount may be usually about 0.1 to 5000 mg, and preferably 1 to 1200 mg, per day for an adult, and it is preferable that this amount is administered once or in about 2 to 4 divided portions in one day.

### EXAMPLES

In the following, the raw material compounds used in the present invention, comparative compounds, and the method for producing the compound of the present invention will be described in detail with reference to Synthesis Examples and Examples. In addition, the HPLC analysis was performed under the following conditions.

Column: Inertsil ODS-3 150 mm × 4.6 mm ID
Eluent: 0.05 M KH₂PO₄/acetonitrile = 50/50 (v/v)
Flow rate: 1.0 mL/min
Column temperature: 40°C
Amount of injection: 2 µL
Wavelength of detection: 254 nm

### Synthesis Example 1:

### 4-(5-hydroxypentyloxy)-2,3-diniethylpyridine-N-oxide

Under a nitrogen stream and in a silicone oil bath, 140 mL of 1,5-pentanediol was introduced, and while stirring, 4.6 g (0.2 mol, 2.0 eq.) of metallic sodium (Na) was added. Subsequently, the silicone oil bath was heated, and the mixture was allowed to react at 100°C for 1 hour. To the obtained reaction liquor, 15.8 g (1.0 mol, 1.0 eq.) of 4-chloro-2, 3-dimethylpyri dien-N-oxide was added, and then the mixture was allowed to react at an elevated temperature of 120°C for 2 hours. The reaction liquor was cooled, and then concentrated under reduced pressure and dried to solid, to obtain 53.3 g of concentrated residue, and the concentrated residue was purified using a silica gel column to obtain 25.0 g of 4- (5-hydroxypentyloxy) -2, 3-dimethylp yridine-N-oxide.

### Synthesis Example 2:

### 4-(5-hydroxypentyloxy)-2-acetoxymethyl-3-methylpyridine

To 24.5 g (0.1 mol, 1.0 eq.) of 4-(5-hydroxypentyloxy) -2,3-dimethylpyridine-N-oxide, 153.1 g (1.5 mol, 15 eq.) of acetic anhydride was added, and the mixture was allowed to react at 100°C for 5 hours. Acetic anhydride was distilled off, and then the obtained concentrated residue was purified using a silica gel column, to obtain 12.1 g of 4-(5-hydroxypentyloxy)-2-acetoxymethyl-3-methylpyridine (yield 39.2 %).

### Synthesis Example 3:

### 4-(5-hydroxypentyloxy)-2-hydroxymethyl-3-methylpyridine

11. 8 g (0.038 mol, 1.0 eq.) of 4- (5-hydroxypentyloxy) -2-acetoxymethyl-3-methylpyridine was added dropwise to 24.4 g (0.152 mol, 4.0 eq.) of a 20% aqueous solution of sodium hydroxide, and the mixture was allowed to react at room temperature for 1 hour. Then, the reaction mixture was extracted with 150 mL of chloroform, dried over magnesium sulfate, and then concentrated and dried to solid, to obtain 6.8 g of 4-(5-hydroxypentyloxy)-2-hydroxymethyl-3-methylpyridine as pale yellow crystals (yield 79.1%).

### Synthesis Example 4:

### 4-(6-hydroxyhexyloxy)-2,3-dimethylpyridine-N-oxide

Under a nitrogen stream and in a silicone oil bath, 47.3 g (0.4 mol, 4.0 eq.) of 1,6-hexanediol and 100 mL of toluene were introduced, and while stirring, the mixture was dissolved at 55°C. Then, 4.6 g (0.2 mol, 2.0 eq.) of metallic Na was added in small portions over 2 hours. The temperature increased from 79°C to 91°C. Subsequently, the silicone oil bath was heated, and the mixture was allowed to react at 100°C for 1 hour. To the obtained reaction liquor, 15.8 g (0.1 mol, 1.0 eq.) of 4-chloro-2,3-dimethylpyridine-N-oxide was added, then the temperature was elevated up to 110°C, and the mixture was allowed to react for 2 hours. The reaction liquor was cooled by standing overnight, and then the separated toluene layer was removed by decantation. To the resulting residue, 100 mL of methanol was added, and the mixture was stirred. The insoluble was removed by filtration, and then the obtained filtrate was concentrated under reduced pressure and dried to solid, to obtain 69.2 g of 4-(6-hydroxyhexyloxy)-2,3-dimethylpyridine-N-oxide.

### Synthesis Example 5:

### 4-(6-hydroxyhexyloxy)-2-acetoxymethyl-3-methylpyridine

To 68.2g (0.1 mol, 1.0 eq.) of 4-(6-hydroxyhexyloxy)-2,3-dimethylpyridine-N-oxide, 153.1 g (1.5 mol, 15 eq.) of acetic anhydride was added, and the mixture was allowed to react at 100°C for 4 hours. Acetic anhydride was distilled off, and then 96.3 g of the resulting concentrated residue was purified using a silica gel column (chloroform:methanol = 40:1), to obtain 30.7 g of 4-(6-hydroxyhexyloxy)-2-acetoxymethyl-3-methylpyridine as an orange-colored oily matter (yield 95.0%).

### Synthesis Example 6:

### 4-(6-hydroxyhexyloxy)-2-hydroxymethyl-3-methylpyridine

29.7 g (0.092 mol, 1.0 eq.) of 4-(6-hydroxyhexyloxy)-2-acetoxymethyl-3-methyl-4-pyridine was added dropwise to 147 g (0.736mol, 8.0eq.) of a 20% aqueous solution of sodiumhydroxide, and the mixture was allowed to react at room temperature for 1 hour. Then, the reaction mixture was extracted with 200 mL of chloroform, dried over magnesium sulfate, and then concentrated and dried to solid, to obtain 22.7 g of an orange-brown oily matter. Since it was confirmed that some starting raw materials did not undergo hydrolysis, the product was further allowed to react for 3 hours at room temperature in a 20% aqueous solution of sodium hydroxide (12.0 eq.), and then the reaction liquor was extracted with 150 mL of chloroform, and concentrated under reduced pressure. Then, 19.0 g of the resulting brown oilymatter was purified using a silica gel column (chloroform), to obtain 7.1 g of 4- (6-hydroxyhexyloxy)-2-hydro xymethyl-3-methylpyridine as an oily matter (yield 58.2%).

### Synthesis Example 7:

### 4-(7-hydroxyheptyloxy)-2,3-dimethylpyridine-N-oxide

Under a nitrogen stream and in a silicone oil bath, 24.8 g (0.188 mol, 2.2 eq.) of 1, 7-heptanediol and 100 mL of toluene were introduced, and while stirring, the mixture was dissolved at 60°C. Then, 3.1 g (0.136 mol, 1.6 eq.) of metallic Na was added. Subsequently, the silicone oil bath was heated, and the mixture was allowed to react at 100°C for 1 hour. To the obtained reaction liquor, 13.4 g (0.085 mol, 1.0 eq.) of 4-chloro-2,3 -dimethylpyridine-N-oxide was added, then the temperature was elevated to 100°C, and the mixture was allowed to react for 2 hours. The reaction liquor was cooled by standing overnight, and the separated toluene layer was removed by decantation. To the resulting residue, 100 mL of methanol was added, and the mixture was stirred. The insoluble was removed by filtration, and then the obtained filtrate was concentrated under reduced pressure and dried to solid, to obtain 45.5 g of 4-(7-hydroxy heptyloxy)-2,3-dimethylpyridine-N-oxide.

### Synthesis Example 8:

### 4-(7-hydroxyheptyloxy)-2-acetoxymethyl-3-methylpyridine

To 44.5 g (0.085 mol, 1.0 eq.) of 4- (7-hydroxyheptyloxy)-2,3-dimethylpyridine-N-oxide, 130.2 g (1.275 mol, 15 eq.) of acetic anhydride was added, and the mixture was allowed to react at 100°C for 4 hours. Acetic anhydride was distilled off, and then 61.7 g of the resulting concentrated residue was purified using a silica gel column (chloroform:methanol =40:1), to obtain 26.3 g of 4-(7-hydroxyheptyloxy)-2-acetoxymethyl-3-methylpyri dine as an oily matter (yield 91.6%).

### Synthesis Example 9:

### 4-(7-hydroxyheptyloxy)-2-hydroxymethyl-3-methylpyridine

25.3 g (0.075 mol, 1.0 eq.) of 4-(7-hydroxyheptyloxy)-2-acetoxymethyl-3-methylpyridine was added dropwise to 120 g (0.6 mol, 8.0 eq.) of a 20% aqueous solution of sodium hydroxide, and the mixture was allowed to react at room temperature for 1.5 hours. Then, the reaction liquor was extracted with 200 mL of chloroform, dried over magnesium sulfate, and then concentrated and dried to solid, to obtain 14.6 g of 4-(7-hydroxyheptyloxy)-2-hydroxymethyl-3-methylpyridine as a pale brown oily matter (yield 76.8%).

### Synthesis Example 10:

### 4-(8-hydroxyoctyloxy)-2,3-dimethylpyridine-N-oxide

Under a nitrogen stream and in a silicone oil bath, 47.4 g (0.324 mol, 3.6 eq.) of 1,8-octanediol and 100 mL of toluene were introduced, and while stirring, the mixture was dissolved at 60°C. Then, 4.1 g (0.18 mol, 2.0 eq.) of metallic Na was added. Subsequently, the silicone oil bath was heated, and the mixture was allowed to react at 100°C for 1 hour. To the obtained reaction liquor, 14.2 g (0.09 mol, 1.0 eq.) of 4-chloro-2, 3-dime thylpyridine-N-oxide was added, then the temperature was elevated to 110°C, and the mixture was allowed to react for 2 hours. The reaction liquor was cooled by standing overnight, and the separated toluene layer was removed by decantation. To the resulting residue, 150 mL of methanol was added, and the mixture was stirred. The insoluble was removed by filtration, and then the obtained filtrate was concentrated under reduced pressure and dried to solid, to obtain 68.1 g of 4-(8-hydroxyoctyloxy)-2,3-dimethylpyridine-N-oxide.

### Synthesis Example 11:

### 4-(8-hydroxyoctyloxy)-2-acetoxymethyl-3-methylpyridine

To 67.1 g (0.09mol, 1.0 eq.) of 4-(8-hydroxyoctyloxy) -2,3-dimethylpyridine-N-oxide, 137.8 g (1.35 mol, 15 eq.) of acetic anhydride was added, and the mixture was allowed to react at 100°C for 4 hours. Acetic anhydride was distilled off, and then 98.5 g of the resulting concentrated residue was purified using a silica gel column (chloroform:methanol = 40:1), to obtain 41. 6 g of 4-(8-hydroxyoctyloxy)-2-acetoxymethyl-3-methylpyridine as an oily matter.

### Synthesis Example 12:

### 4-(8-hydroxyoctyloxy)-2-hydroxymethyl-3-methylpyridine

40.6g (0.09mol, 1.0eq.) of 4-(8-hydroxyoctyloxy)-2-acet oxymethyl-3-methylpyridine was added dropwise to 144 g (0.72 mol, 8.0 eq.) of a 20% aqueous solution of sodium hydroxide, and the mixture was allowed to react at room temperature for 1 hour. Then, the reaction liquor was extracted with 200 mL of chloroform, dried over magnesium sulfate, and then concentrated and dried to solid, to obtain 28.0 g of 4-(8-hydroxyoctyloxy)-2-hydroxymethyl-3-methylpyridine as an orange-brown oily matter.

### Synthesis Example 13:

### 4-(9-hydroxynonyloxy)-2,3-dimethylpyridine-N-oxide

Under a nitrogen stream and in a silicone oil bath, 50.0 g (0.312 mol, 4.0 eq.) of 1, 9-nonanediol and 86.7 mL of toluene were introduced, and while stirring under a nitrogen stream, the mixture was heated to 67°C. Then, 3.6 g (0.156 mol, 2.0 eq.) of metallic Na was added. Subsequently, the silicone oil bath was heated, and the mixture was allowed to react at 100°C for 1 hour. To the obtained reaction liquor, 12.3 g (0.078 mol, 1.0 eq.) of 4-chloro-2,3-dimethylpyridine-N-oxide was added, then the temperature was elevated to 109°C, and the mixture was allowed to react for 5 hours. The reaction liquor was cooled by standing overnight, and the separated toluene layer was removed by decantation. To the resulting residue, 130 mL of methanol was added, and the mixture was stirred. The insoluble was removed by filtration, and then the obtained filtrate was concentrated under reduced pressure and dried to solid, to obtain 56.4 g of 4-(9-hydroxynonyloxy)-2,3-dimethylpyridine-N-oxide as an oily matter.

### Synthesis Example 14:

### 4-(9-hydroxynonyloxy)-2-acetoxymethyl-3-methylpyridine

To 56.4 g (0.078 mol, 1.0 eq.) of 4-(9-hydroxynonyloxy)-2,3-dimethylpyridine-N-oxide, 119.3 g (1.169 mol, 15 eq.) of acetic anhydride was added, and the mixture was allowed to react at 100°C for 5 hours. Acetic anhydride was distilled off, and then 91.7 g of the resulting concentrated residue was purified using a silica gel column (chloroform:methanol = 40:1), to obtain 34.7 g of 4-(9-hydroxynonyloxy)-2-acetoxymethyl-3-methylpyri dine as an oily matter.

### Synthesis Example 15:

### 4-(9-hydroxynonyloxy)-2-hydroxymethyl-3-methylpyridine

34.7 g (0.078 mol, 1.0 eq.) of 4-(9-hydroxynonyloxy)-2-acetoxymethyl-3-methylpyridine was added dropwise to 125 g (0.625mol, 8.0eq.) of a 20% aqueous solution of sodiumhydroxide, and the mixture was allowed to react at room temperature for 1 hour. Then, the reaction liquor was extracted with 173 mL of chloroform, dried over magnesium sulfate, and then concentrated and dried to solid, to obtain 28.4 g of 4-(9-hydroxynonyloxy)-2-hydroxymethyl-3-methylpyridine as a brown oily matter.

### Synthesis Example 16:

### 4-(10-hydroxydecyloxy)-2,3-dimethylpyridine-N-oxide

Under a nitrogen stream and in a silicone oil bath, 50.0 g (0.287 mol, 4.0 eq.) of 1,10-decanediol and 79.7 mL of toluene were introduced, and while stirring under a nitrogen stream, the mixture was heated to 67°C. Then, 3.3 g (0.143 mol, 2.0 eq.) of metallic Na was added. Subsequently, the silicone oil bath was heated, and the mixture was allowed to react at 100°C for 1 hour. To the obtained reaction liquor, 11.3 g (0.072 mol, 1.0 eq.) of 4-chloro-2,3-dimethylpyridine-N-oxide was added, then the temperature was elevated to 109°C, and the mixture was allowed to react for 3 hours. The reaction liquor was cooled by standing overnight, and the separated toluene layer was removed by decantation. To the resulting residue, 120 mL of methanol was added, and the mixture was stirred. The insoluble was removed by filtration, and then the obtained filtrate was concentrated under reduced pressure and dried to solid, to obtain 72.9g of 4-(10-hydroxydecyloxy)-2,3-dimethylpyridine-N-oxide as a brown oily matter.

### Synthesis Example 17:

### 4-(10-hydroxydecyloxy)-2-acetoxymethyl-3-methylpyridine

To 72.9 g (0.072 mol, 1.0eq.) of 4- (10-hydroxydecyloxy)-2, 3-dimethylpyridine-N-oxide, 109. 7 g (1.075mol,15eq.) of acetic anhydride was added, and the mixture was allowed to react at 100°C for 4 hours. Acetic anhydride was distilled off, and then 93.5 g of the resulting concentrated residue was purified using a silica gel column (chloroform:methanol = 40:1), to obtain 18.5 g of 4-(10-hydroxydecyloxy)-2-acetoxymethyl-3-methylpyridine as a mixture containing unreacted raw materials, as an orange-brown oily matter. To 18.5 g of the orange-brown oily matter, 100. 0 g (1.021 mol, 15 eq.) of acetic anhydride was added, and the mixture was allowed to react at 100°C for 3 hours. The reaction liquor was concentrated under reducedpressure and dried to solid, to obtain 20.4 g of 4- (10-hydroxydecyloxy) -2-acetoxy methyl-3-methylpyridine.

### Synthesis Example 18:

### 4-(10-hydroxydecyloxy)-2-hydroxymethyl-3-methylpyridine

14.0g (0. 037 mol, 1.0 eq.) of 4-(10-hydroxydecyloxy)-2-acetoxymethyl-3-methylpyridine was added dropwise to 59 g (0.296 mol, 8.0 eq.) of a 20% aqueous solution of sodium hydroxide, and the mixture was allowed to react overnight at room temperature. Then, the reaction liquor was extracted with 100mL of chloroform, dried over magnesium sulfate, and then concentrated and dried to solid, to obtain 12.0 g of 4- (10-hydroxydecyloxy) -2-hydroxy methyl-3-methylpyridine as an orange-brown oily matter.

### Example 1: 2-[{4-(5-hydroxypentyloxy)-3-methylpyridin-2-yl}methylthio] -1H-benzimidazole

In a nitrogen atmosphere, 6.5 g (0.029 mol, 1.0 eq.) of 4-(5-hydroxypentyloxy)-2-hydroxymethyl-3-methylpyridine was dissolved in 100 mL of chloroform, and while conducting salt-ice cooling, the solution was added to a solution prepared by dissolving 10.4 g (0.087 mol, 3 eq.) of thionyl chloride in 90 mL of chloroform. The mixture was allowed to react at -10°C for 3 hours. The reaction liquor was adjusted to pH 9 using a saturated aqueous solution of sodium carbonate, and then the resultant was extracted with 90 mL of chloroform, dried over magnesium sulfate, then concentrated and dried to solid, to obtain7.8g of 4-(5-hydroxypentyloxy)-2-chloromethyl-3-methyl pyridine. To a solution prepared by adding 7.5 g (0.029 mol, 1.0 eq.) of 4-(5-hydroxypentyloxy)-2-chloromethyl-3-methylp yridine and 3.5 g (0.023 mol, 0.8 eq.) of 2-mercaptobenzimidazole with cooling in ice water and stirring, a solution of 1. 4 g (0. 035 mol, 1.2 eq.) of NaOH in 60 mL of ethanol was added over 1.5 hours. Subsequently, the temperature was elevated to 50°C, and the mixture was allowed to react for 15 minutes, and then concentrated under reduced pressure, to obtain 12.8 g of an orange-colored oily matter. The concentrated residue was purified using a silica gel column (chloroform:methanol =40:1), to obtain 4.8 g of an orange-colored oily matter. The oily matter was recrystallized from ethyl acetate:methanol = 20:1 (21 vol), to obtain 2.8 g of colorless crystals of 2- [{4-(5-hydroxypentyl oxy)-3-methylpyridin-2-yl}methylthio]-1H-benzimidazole (HPLC: 99.4 Area%, yield 26.9%).
¹H-NMR (400 MHz, CDCl₃) δ: 1.47-1.73 (7H, m), 2.25 (3H, s), 3.70 (2H, t J=6 Hz), 4.03 (2H, t J=6 Hz), 4.37 (2H, s), 6.72 (1H, d J=6 Hz), 7.08-7.24 (2H, m), 7.33-7.85 (2H, m), 8.33 (1H, d J=6 Hz), 12.52-13.43 (1H, bs)
MS m/z: 357 (M⁺)

### Example 2:

### 2-[{4-(6-hydroxyhexyloxy)-3-methylpyridin-2-yl}methylthio]-1H-benzimidazole

In a nitrogen atmosphere, 6.9 g (0.029 mol, 1.0 eq.) of 4-(6-hydroxyhexyloxy)-2-hydroxymethyl-3-methylpyridine was dissolved in 120 mL of dichloromethane, and a solution prepared by dissolving 10.4 g (0.087 mol, 3 eq.) of thionyl chloride in 60 mL of dichloromethane was added thereto. The mixture was allowed to react at -10°C for 1.5 hours. The reaction liquor was adjusted to pH 8 using a saturated aqueous solution of sodium carbonate, and then the dichloromethane layer was dried over magnesium sulfate, then concentrated and dried to solid, to obtain 6.7 g of 4-(6-hydroxyhexyloxy)-2-chloromethyl-3-methyl pyridine as an orange-colored oily matter (yield 89.3%). To a solution prepared by adding 6.5 g (0.025 mol, 1.0 eq.) of 4-(6-hydroxyhexyloxy)-2-chloromethyl-3-methylpyridine and 3.5 g (0.023 mol, 0.8 eq.) of 2-mercaptobenzimidazole with cooling in ice water and stirring, a solution of 1.4 g (0.035 mol, 1.2 eq.) of NaOH in 60 mL of ethanol was added over 1.5 hours. Subsequently, the temperature was elevated to 50°C, and the mixture was allowed to react for 15 minutes, and then concentrated under reduced pressure, to obtain 12.8 g of an orange-colored oily matter. The concentrated residue was purified using a silica gel column (chloroform:methanol = 40:1), to obtain 4.8 g of an orange-colored oily matter. The oilymatter was recrystallized from ethyl acetate:methanol =20:1 (21 vol), to obtain 2.8 g of colorless crystals of 2-[{4-(5-hydroxyhex yloxy)-3-methylpyridin-2-yl)methylthio]-1H-benzimidazole (HPLC: 99.4 Area%, yield 26.9%).
¹H-NMR (400 MHz, CDCl₃) δ: 1.34-1.99 (9H, m), 2.26 (3H, s), 3. 67 (2H, t J=6 Hz), 4.03 (2H, t J=6 Hz), 4.37 (2H, s), 6.73 (1H, d J=6 Hz), 7.11-7.24 (2H, m), 7.33-7.77 (2H, m), 8.34 (1H, d J=6 Hz), 12.69-13.34 (1H, bs)
MS m/z: 371 (M⁺)

### Example 3:

### 2-[{4-(7-hydroxyheptyloxy)-3-methylpyridin-2-yl}methylthio] -1H-benzimidazole

8.1 g (0.032 mol, 1.0 eq.) of 4-(7-hydroxyheptyloxy)-2-hydroxymethyl-3-methylpyridine was dissolved in 120 mL of dichloromethane, and 11.4 g (0.096 mol, 3.0 eq.) of thionyl chloride was added. The mixture was allowed to react at -10°C for 2 hours. The reaction liquor was adjusted to pH 8 using a saturated aqueous solution of sodium carbonate, and then the dichloromethane layer was dried over magnesium sulfate, then concentrated and dried to solid, to obtain 8.7 g of 4- (7-hydroxy heptyloxy)-2-chloromethyl-3-methylpyridine as an orange-brown oily matter.

To a solution prepared by dissolving and stirring 3.6 g (0. 024 mol, 0.8 eq.) of 2-mercaptobenzimidazole and 6. 9 g (0.036 mol, 1.2 eq.) of 28% sodium methoxide in 100 mL of methanol, a solutionpreparedby dissolving 8.2 g of 4-(7-hydroxyheptyloxy) -2-chloromethyl-3-methylpyridine in 100 mL of methanol was added at 27°C. Subsequently, the mixture was heated to reflux for 30 minutes, cooled and then concentrated under reduced pressure, to obtain 9.0 g of an orange-colored oily matter. 200 mL of ethyl acetate and 12 g of methanol were added to dissolve the concentrated residue, then 150 mL of water was added, and the organic layer was washed with water. The aqueous layer was extracted with 50 mL of ethyl acetate, and then the organic layer was combined. 27 g of silica gel was added to the combined organic layer, the mixture was stirred for 30 minutes, and then the silica gel was removed by filtration. The filtrate was concentrated under reduced pressure, to obtain 8.4 g of orange-white crystals. The crystals were suspended in 126 g (15 vol) of ethyl acetate, and the suspension was heated to 57°C. Then, 2.0 g of methanol was added to completely dissolve the crystals. Crystals were precipitated by standing the solution to cool. After aging at 20°C for 30 minutes, the crystals were collected by filtration, and then the crystals were dried under reducedpressure, to obtain 4.0 of colorless crystals of 2-[{4-(7-hydroxyheptyloxy)-3-methylpyridin-2-yl}methylthio]-1H-benzimidazole (HPLC purity: 98.2 Area%, yield 34.5%).
¹H-NMR (400 MHz, CDCl₃) δ: 1.30-1.99 (11H, m), 2.25 (3H, s), 3.66 (2H, t J=6 Hz), 4.02 (2H, t J=6 Hz), 4.37 (2H, s), 6.72 (1H, d J=6 Hz), 7.10-7.25 (2H, m), 7.31-7.81 (2H, m), 8.33 (1H, d J=6 Hz), 12.61-13.45 (1H, bs)
MS m/z: 385 (M⁺)

### Example 4:

### 2-[{4-(8-hydroxyoctyloxy)-3-methylpyridin-2-yl}methylthio]-1H-benzimidazole

27.0 g (0.09 mol, 1.0 eq.) of 4-(8-hydroxyoctyloxy)-2-hydroxymethyl-3-methylpyridine was dissolved in 140 mL of dichloromethane, and 21.4 g (0.18 mol, 2.0 eq.) of thionyl chloride was added. The mixture was allowed to react at -10°C for 3.5 hours. The reaction liquor was adjusted to pH 8 using 300 g of a saturated aqueous solution of sodium carbonate, and then the dichloromethane layer was dried over magnesium sulfate, then concentrated and dried to solid, to obtain 25.2 g of 4-(8-hydroxyoctyloxy)-2-chloromethyl-3-methylpyridine as an orange-brown oily matter (yield 98.1%).

To a solution prepared by dissolving and stirring 4.8 g (0.032 mol, 0.8 eq.) of 2-mercaptobenzimidazole and 9.3 g (0.048 mol, 1.2 eq.) of 28% sodium methoxide in 100 mL of methanol, a solution prepared by dissolving 11.4 g of 4-(8-hydroxyoctyloxy) -2-chloromethyl-3-methylpyridine in 60 mL of methanol was added at 27°C. Subsequently, the mixture was heated to reflux for 30 minutes, cooled and then concentrated under reduced pressure, to obtain 22.8 g of an orange-colored oily matter. The concentrated residue was dissolved in 250 mL of ethyl acetate, and then the organic layer was washed with 200 mL of water. The organic layer was left to stand overnight, and then crystals were precipitated. Thus, 20 mL of methanol was added, and the mixture was heated to 42°C to dissolve. Then, 27.6 g of silica gel was added, the mixture was stirred for 30 minutes, and then the silica gel was removed by filtration. The filtrate was concentrated under reduced pressure, to obtain 9.0 g of orange-white crystals. The crystals were suspended in 135 g (15 vol) of ethyl acetate, the suspension was heated to 57°C, and then 12.8 g of methanol was added to completely dissolve the crystals. Crystals were precipitated by standing the solution to cool. After aging at 20°C for 1 hour, the crystals were collected by filtration. The crystals were dried under reduced pressure, to obtain 3.7 g of colorless crystals of 2-[{4-(8-hydroxyoctyloxy)-3-methylpyridin-2-yl}methylthio]-1H-benzimidazole (HPLC purity: 98.4 Area%, yield 23.1%).

¹H-NMR (400 MHz, CDCl₃) δ: 1.10-1.95 (13H, m), 2.26 (3H, s), 3.65 (2H, t J=6 Hz), 4.02 (2H, t J=6 Hz), 4.37 (2H, s), 6.73 (1H, d J=6 Hz), 7.11-7.24 (2H, m), 7.44-7.64 (2H, m), 8.33 (1H, d J=6 Hz), 12.26-13.84 (1H, bs)
MS m/z: 399 (M⁺)

### Example 5:

### 2-[{4-(9-hydroxynonyloxy)-3-methylpyridin-2-yl}methylthio]-1H-benzimidazole

28.4 g (0.078 mol, 1.0 eq.) of 4-(9-hydroxynonyloxy)-2-hydroxymethyl-3-methylpyridine was dissolved in 121 mL of dichloromethane, and a solution of 18.5 g (0.155 mol, 2.0 eq.) of thionyl chloride in 69 mL dissolved solution of dichloromethane was added. The mixture was allowed to react at -17°C for 3 hours. The reaction liquor was adjusted to pH 9 using a saturated aqueous solution of sodium carbonate, and then the extracted dichloromethane layer was dried over magnesium sulfate, then concentrated and dried to solid, to obtain 17.2 g of 4-(9-hydroxynonyloxy)-2-chloromethyl-3-methylpyridine (yield 74.1%).

To a solution prepared by dissolving and stirring 7.2 g (0.048 mol, 0.6 eq.) of 2-mercaptobenzimidazole and 14.0 g (0.073 mol, 0.93 eq.) of 28% sodium methoxide in 181 mL of methanol, 17.2 g (0.057 mol, 1.0 eq.) of 4-(9-hydroxynonyloxy)-2-chloro methyl-3-methylpyridine was added at 27°C. Subsequently, the mixture was heated to reflux for 30 minutes, cooled and then concentrated under reduced pressure, to obtain an orange-colored oily matter. The concentrated residue was dissolved in 377 mL of ethyl acetate, and then the organic layer was washed with 302 mL of water. Since the organic layer had crystals precipitated, 30 mL of methanol was added, and the mixture was heated to 35°C to dissolve. Then, 41. 6 g of silica gel was added, the mixture was stirred for 30 minutes, and then silica gel was removed by filtration. The filtrate was concentrated under reduced pressure, to obtain 13.9 g of an orange-colored oily matter. The oily matter was heated in 208.5 g (15 vol) of ethyl acetate to dissolve, and then crystals were precipitated by standing the solution to cool. After aging at 20°C for 1 hour, the crystals were collected by filtration. The crystals were dried under reduced pressure, to obtain 4.3 g of pale yellow crystals of 2-[{4-(9-hydroxynonyloxy)-3-methylpyridin-2-yl}m ethylthio]-1H-benzimidazole (HPLC purity: 96.4 Area%, yield 17.7%).
¹H-NMR (400 MHz, CDCl₃) δ: 1.19-1.90 (15H, m), 2.26 (3H, s), 3.64 (2H, t J=6 Hz), 4.02 (2H, t J=6 Hz), 4.37 (2H, s), 6.73 (1H, d J=6 Hz), 7.11-7.24 (2H, m), 7.36-7.66 (2H, m), 8.33 (1H, d J=6 Hz), 12.40-13.60 (1H, bs)
MS m/z: 413 (M⁺)

### Example 6:

### 2-[{4-(10-hydroxydecyloxy)-3-methylpyridin-2-yl}methylthio] -1H-benzimidazole

11.5 g (0.035 mol, 1.0 eq.) of 4-(10-hydroxydecyloxy)-2-hydroxymethyl-3-methylpyridine was dissolved in 120 mL of dichloromethane, and a solution of 12.5 g (0.105 mol, 3.0 eq.) of thionyl chloride in 60 mL dissolved solution of dichloromethane was added. The mixture was allowed to react at -17 to -12°C for 3 hours. The reaction liquor was adjusted to pH 8 using a saturated aqueous solution of sodium carbonate, and then the extracted dichloromethane layer was dried over magnesium sulfate, then concentrated and dried to solid, to obtain 10.6 g of 4-(10-hydroxydecyloxy)-2-chloromethyl-3-me thylpyridine (yield 96.4%).

To a solution prepared by dissolving 4.3 g (0.029 mol, 0.9 eq.) of 2-mercaptobenzimidazole and 4.3 g (0.029 mol, 0.9 eq.) of 4-(10-hydroxydecyloxy)-2-chloromethyl-3-methylpyrid ine and 200 mL of methanol, 6.8 g (0.035 mol, 1.1 eq.) of 28% sodium methoxide was added at 22°C. Subsequently, the mixture was heated to reflux for 30 minutes, cooled and then concentrated under reduced pressure, to obtain 18.0 g of an orange-colored oily matter. The concentrated residue was dissolved in a liquid mixture of 250 mL of ethyl acetate and 2 mL of methanol, and then the organic layer was washed with 200 mL of water. 18.0 g of silica gel was added to the organic layer, the mixture was stirred for 30 minutes, and then the silica gel was removed by filtration. The filtrate was concentrated under reduced pressure, to obtain 13.4 g of yellow-white crystals. The crystals were suspended in 201 g (15 vol) of ethyl acetate, the suspension was heated to 55°C, and then 17.5 g of methanol was added to completely dissolve the crystals. Crystals were precipitated by standing the solution to cool. After aging at 10 to 15°C for 30 minutes, 6.5 g of the crystals were collected by filtration. 280 g (43-fold amount) of methanol was added to the crystals, and the mixture was heated with stirring. The insoluble was removed by filtration, and then the filtrate was concentrated under reduced pressure and dried, to obtain 6.7 g of pale yellow crystals. The crystals were dissolved in 134 g (20-fold amount) of ethyl acetate and 6.7 g of methanol by heating to 60°C, and then the solution was cooled by standing. After stirring overnight at 201°C, the crystals were collected by filtration, and dried under reduced pressure, to obtain 5.0 g of colorless crystals of 2-[{4-(10-hydroxydecyloxy) -3-methyl pyridin-2-yl}methylthio]-1H-benzimidazole (HPLC purity: 96.3 Area%, yield 36.5%).

¹H-NMR (400 MHz, CDCl₃) δ: 1.10-1.95 (17H, m), 2.26 (3H, s), 3.64 (2H, t J=6 Hz), 4.02 (2H, t J=6 Hz), 4.37 (2H, s), 6.73 (1H, d J=6 Hz), 7.11-7.24 (2H, m), 7.44-7.64 (2H, m), 8.33 (1H, d J=6 Hz), 12.30-13.68 (1H, bs)
MS m/z: 427 (M⁺)

### Pharmacological Test Example 1

### Antibacterial Power Test

### (Method)

An in vitro test was performed in a Columbia agar medium using a standard strain of *H. pylori,* ATCC 43504. The strain was cultured in a Columbia agar medium at 37°C and pH 7.0 for 3 days, and on the 4^{th} day, the minimum inhibitory concentration (MIC, µg/ml) was determined. Each of the test objects was dissolved in a 1% DMSO solution. Furthermore, as antibiotic control drugs, ampicillin from the penicillin family (control drug 1), gentamycin from the aminoglycoside family (control drug 2), tetracycline from the tetracycline family (control drug 3), and ofloxacin from the new quinolone family (control drug 4) were used.

### (Results)

The results as the in vitro anti-*Helicobacter pylori* activity (MIC (µg/ml)) are presented in Table 1.

**[Table 1]**

| In vitro anti-*Helicobacter pylori* activity of novel pyridine derivatives | |
|---|---|
| Test object | MIC (µg/ml) |
| Example 1 | 0.3 |
| Example 2 | 0.1 |
| Example 3 | 0.1 |
| Example 4 | 0.03 |
| Example 5 | 0.1 |
| Example 6 | 0.3 |
| Comparative Compound 1 | 10.0 |
| Comparative Compound 2 | 3.0 |
| Control Drug 1 (Ampicillin) | 0.1 |
| Control Drug 2 (Gentamycin) | 0.3 |
| Control Drug 3 (Tetracycline) | 0.3 |
| Control Drug 4 (Ofloxacin) | 1.0 |

As a result of the present test, the compounds of the present invention (Examples 1 to 6) were acknowledged to have strong antibacterial effects against *H. pylori,* as equivalently strong as the various antibacterial agents (control drugs 1 to 4).

As for the anti-*Helicobacterpylori* activity (MIC (µg/ml), it was found that the compounds of the present invention showed anti-*Helicobacter pylori* activities that were clearly 10 times or more stronger than the activities of the existing similar two compounds (comparative compounds 1 and 2). In addition, the compounds of Comparative Examples are the above-mentioned 2-[{4-(2-hydroxyethoxy)-3-methylpyridin-2-yl}methylthio]-1H -benzimidazole (comparative compound 1) and 2-[{4-(3-hydroxy propoxy)-3-methylpyridin-2-yl}methylthio]-1H-benzimidazole (comparative compound 2).

### Pharmacological Test Example 2

### (Method)

An in vitro test was performed in a Columbia agar medium using standard strains of *H. pylori,* NCTC 11637 and 11916, clinical isolates PT#1045482, PT#1045483 and PT#1045484, and ofloxacin- and roxithromycin-resistant, clinical isolates TY2, 4 and 5. Each of the test objects was dissolved in a 1% DMSO solution. Furthermore, as antibiotic control drugs, roxithromycin from the macrolide family and ofloxacin from the new quinolone family were used. The strains were cultured at 37°C and pH 7.0 for 3 days, and on the 4^{th} day, the minimum inhibitory concentrations (MIC, µg/ml) were determined.

### (Results)

The results as the effect (MIC (µg/ml)) on the resistant bacteria of *Helicobacterpylori* in vitro are presented in Table 2.

**[Table 2]**

| Effect of novel pyridine derivatives on resistant bacteria of *Helicobacter pylori* | | | | | | | | | |
|---|---|---|---|---|---|---|---|---|---|
| | Bacterial species | Example 1 | Example 2 | Example 3 | Example 4 | Example 5 | Example 6 | RXM | OFLX |
| Standard strain | NCTC 11637 | 0.3 | 0.1 | 0.1 | 0.03 | 0.1 | 0.3 | 0.2 | 0.8 |
| | NCTC 11916 | 0.3 | 0.1 | 0.1 | 0.03 | 0.1 | 0.2 | 0.1 | 0.8 |
| Clinical isolate | PT#1045482 | 0.2 | 0.1 | 0.1 | 0.03 | 0.2 | 0.3 | 0.4 | 0.8 |
| | PT#1045483 | 0.3 | 0.1 | 0.1 | 0.03 | 0.1 | 0.3 | 0.2 | 0.8 |
| | PT#1045484 | 0.3 | 0.3 | 0.1 | 0.03 | 0.1 | 0.3 | 0.2 | 0.4 |
| Resistant strain | TY2 | 0.2 | 0.3 | 0.1 | 0.03 | 0.1 | 0.3 | >100 | 2.5 |
| | TY4 | 0.3 | 0.1 | 0.1 | 0.1 | 0.3 | 0.3 | >100 | 25 |
| | TY5 | 0.3 | 0.1 | 0.1 | 0.03 | 0.2 | 0.3 | 3.5 | 2.5 |

In the Table 2, the various numerical values represent the minimum inhibitory concentration (MIC, µg/ml) of various test objects against various bacterial species, RXM represents roxithromycin, and OFLX represents ofloxacin.

From the results of the present test, the compounds of the present invention (Examples 1 to 6) showed equivalent or stronger antibacterial activities to roxithromycin or ofloxacin compared to the standard strains and clinical isolates. Furthermore, the compounds also exhibited strong antibacterial activities against the roxithromycin- or ofloxacin-resistant clinical isolates. That is, the compounds were acknowledged to exhibit strong antibacterial activities even for the strains that are resistant to roxithromycin from the macrolide family and ofloxacin from the new quinolone family.

The following Table 3 shows the minimum inhibitory concentration (MIC, µg/ml) against the standard strains NCTC11637 and NCTC11916 of the compounds ((a) to (f)) having structures that are similar to the structure of the compound of the present invention. These are data described in Table 6 of JP-A No. 7-69888.

**[Table 3]**

| Effects of existing pyridine derivatives on *Helicobacter pylori* | | | | | | | |
|---|---|---|---|---|---|---|---|
| | Test object | | | | | | |
| Bacterial species | (a) | (b) | (c) | (d) | (e) | (f) | RXM |
| NCTC11637 | 50 | 12.5 | 6.25 | 1.56 | 0.8 | 0.8 | 0.2 |
| NCTC11916 | 50 | 12.5 | 12.5 | 1.56 | 1.56 | 1.56 | 0.1 |

In Table 3, (a) is 5-methoxy-2-(4-methoxy-3,5-dimethyl pyridin-2-yl)methylsulfinyl-1H-benzimidazole, (b) is 5-methoxy-2-(4-methoxy-3,5-dimethylpyridin-2-yl)methylthio-1H-benzimidazole, (c) is 2-[3-methyl-4-(2,2,2-trifluoroetho xy)pyridine-2-yl]methylsulfinyl-1H-benzimidazole, (d) is 2-[3-methyl-4-(2,2,2-trifluoroethoxy)pyridine-2-yl]methylth io-1H-benzimidazole, (e) is 2-[4-(3-methoxypropoxy)-3-methyl pyridin-2-yl]methylsulfinyl-1H-benzimidazole sodium salt, (f) is 2-[4-(3-methoxypropoxy)-3-methylpyridin-2-yl]methylthio-1H-benzimidazole, and RXM is roxithromycin. Various numerical values represent the minimum inhibitory concentration (MIC, µg/ml) of various test obj ects against various bacterial species.

From a comparison of the data of Table 2 and Table 3, it can be seen that the compounds of the present invention (Examples 1 to 6) have activities against the standard strain NCTC11637 that are about 2.7-fold to 1,667-fold, and activities against the strain NCTC11916 that are about 5.2-fold to 1,667-fold, compared to the activities of the compounds of the same class ((a) to (f)). In particular, Example 4 was found to have the strongest activity among the compounds of the same class, and to have an activity as strong as 26. 6-fold against the standard strain NCTC11637 and 52-fold against the standard strain NCTC11916, compared to the activities of the compounds (e) and (f).

### Pharmacological Test Example 3

### (Method)

An in vitro antibacterial test for the compounds of Examples 1 to 6 against various bacteria was performed. As gram-negative bacteria, *Escherichia coli* (ATCC 10536, ATCC 25922), *Klebsiella pneumonia* (ATCC 10031), *Proteus vulgaris* (ATCC 13315), *Pseudomonas aeruginosa* (ATCC 9027), *Salmonella typhimurium* (ATCC 13311), and as gram-positive bacteria, *Staphylococcus aureus*, MRSA (ATCC 33591), *Staphylococcus epidermidis* (ATCC 12228), *Streptococcus pneumonia* (ATCC 6301), *Mycobacterium ranae* (ATCC 110) and *Enterococcus faecalis* (VRE, ATCC 51575) were used. The various bacteria were cultured at 37°C for 20 to 48 hours by conventional methods, and the minimum inhibitory concentrations (MIC, µg/ml) were determined. Each of the test objects were dissolved in a 1% DMSO solution. Furthermore, as an antibiotic control drug, gentamycin (GEM) from the aminoglycoside family was used.

### (Results)

The results as the effects (MIC (µg/ml)) of the compounds on various bacteria in vitro are presented in Table 4.

### [Table 4]

**[Table 4]**

| Effects (µg/ml) of novel pyridine derivatives on gram-negative bacteria and gram-positive bacteria | | | | | | | | |
|---|---|---|---|---|---|---|---|---|
| | Bacterial species | Example 1 | Example 2 | Example 3 | Example 4 | Example 5 | Example 6 | GEM |
| Gram-negative bacteria | Escherichia coli (ATCC 10536) | 100< | 100< | 100< | 100< | 100< | 100< | 0.3 |
| | Escherichia coli (ATCC 25922) | 100< | 100< | 100< | 100< | 100< | 100< | 1.0 |
| | Klebsiella pneumonia (ATCC 10031) | 100< | 100< | 100< | 100< | 100< | 100< | 1.0 |
| | Proteus vulgaris (ATCC 13315) | 100< | 100< | 100< | 100< | 100< | 100< | 0.3 |
| | Pseudomonas aerugionosa (ATCC 9027) | 100< | 100< | 100< | 100< | 100< | 100< | 0.3 |
| | Salmonella typhimurium (ATCC 13311) | 100< | 100< | 100< | 100< | 100< | 100< | 1.0 |
| Gram-positive bacteria | Staphylococcus aureus, MRSA (ATCC 33591) | 100< | 100< | 100< | 100< | 100< | 100< | 1.0 |
| | Staphylococcus epidermidis (ATCC 12228) | 100< | 100< | 100< | 100< | 100< | 100< | 0.1 |
| | Streptococcus pneumonia (ATCC 6301) | 100< | 100< | 100< | 100< | 100< | 100< | - |
| | Mycobacterium ranae (ATCC 110) | 100< | 100< | 100< | 100< | 100< | 100< | 0.3 |
| | Enterococcus faecalis (VRE, ATCC 51575) | 100< | 100< | 100< | 100< | 100< | 100< | - |

As a result of the test, any of the compounds of Examples 1 to 6 was not recognized to have an antibacterial action against various gram-negative bacteria and gram-positive bacteria. On the other hand, gentamycin from the aminoglycoside family exhibited a strong antibacterial action against various gram-negative bacteria and gram-positive bacteria. From this, it was suggested that the compounds of the present invention have no influence at all on the intestinal bacteria.

### Preparation Example 1 Tablets

| | |
|---|---|
| Compound of Example 3 | 50.0 mg |
| Mannitol | 65.5 mg |
| Hydroxypropylcellulose | 2.5 mg |
| Crystalline cellulose | 10.0 mg |
| Corn starch | 10.0 mg |
| Carboxymethylcellulose calcium | 5.0 mg |
| Talc | 2.0 mg |
| Magnesium stearate | 0.2 mg |

Tablets each weighing 145.2 mg were prepared at the above mixing proportions according to a conventional method.

### Preparation Example 2 Granules

| | |
|---|---|
| Compound of Example 4 | 300 mg |
| Lactose | 540 mg |
| Corn starch | 100 mg |
| Hydroxypropylcellulose | 50 mg |
| Talc | 10 mg |

A granular preparation weighing 1000 mg per package was prepared at the above mixing proportions according to a conventional method.

### Preparation Example 3 Capsules

| | |
|---|---|
| Compound of Example 5 | 50 mg |
| Lactose | 15 mg |
| Corn starch | 25 mg |
| Microcrystalline cellulose | 5 mg |
| Magnesium stearate | 1.5 mg |

Capsules each weighing 96.5 mg were produced at the above mixing proportions according to a conventional method.

### Preparation Example 4 Injection

| | |
|---|---|
| Compound of Example 5 | 100 mg |
| Sodium chloride | 3.5 mg |
| Distilled water for injection | Adequate amount |
| (2 ml per one ampoule) | |

An injectable preparation was prepared at the above mixing proportions according to a conventional method.

### Preparation Example 5 Syrup

| | |
|---|---|
| Compound of Example 5 | 200 mg |
| Purified sucrose | 60 g |
| Ethyl parahydroxybenzoate | 5 mg |
| Butyl parahydroxybenzoate | 5 mg |
| Flavor | Adequate amount |
| Colorant | Adequate amount |
| Purified water | Adequate amount |

A syrup preparation was prepared at the above mixing proportions according to a conventional method.

### Preparation Example 6 Tablets

| | |
|---|---|
| Compound of Example 5 | 50 mg |
| Famotidine | 20 mg |
| Cyclodextrin | 26 mg |
| Microcrystalline cellulose | 5 mg |
| Hydroxypropylcellulose | 5 mg |
| Talc | 2 mg |
| Magnesium stearate | 2 mg |

Tablets each weighing 110 mg were prepared at the above mixing proportions according to a conventional method.

### Industrial Applicability

The novel pyridine derivative of the present invention and pharmaceutically acceptable salts thereof are clinically very promising medicines, since the compounds have no effects on the resident bacteria of human being, exhibit specific antibacterial action against *H. pylori*, and also exhibit strong antibacterial activities against strains that are resistant to antibacterial agents.

All of the publications, patents and patent applications cited in the present specification have been directly incorporated into the present specification as reference.

## Claims

1. A pyridine derivative of formula (I): 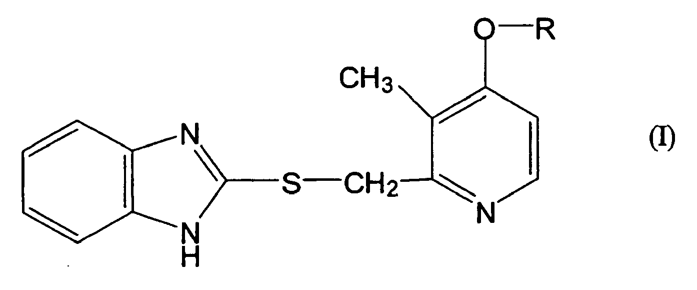 wherein R is a straight-chained hydroxyalkyl group having 5 to 10 carbon atoms, or a pharmaceutically acceptable salt thereof.

2. A derivative according to claim 1 wherein R has a terminal hydroxyl group, or a pharmaceutically acceptable salt thereof,

3. A pyridine derivative according to claim 1 or claim 2 and selected from 2-[{4-(5-hydroxypentyloxy)-3-methylpyridin-2-yl}methylthio]-1H-benzimidzole, 2-[{4-(6-hydroxyhexyloxy)-3-methylpyridin-2-yl}methylthio]-1H-benzimidazole, 2-[{4-(7-hydroxyheptyloxy)-3-methylpyridin-2-yl}methylthio]-1H-benzimidazole, 2-[{4-(8-hydroxyoctyloxy)-3-methylpyridin-2-yl}methylthio]-1H-benzimidazole, 2-[{4-(9-hydroxynonyloxy)-3-methylpyridin-2-yl}methylthio]-1H-benzimidazole, 2-[{4-(10-hydroxydecyloxy)-3-methylpyridin-2-yl}methylthio]-1H-benzimidazole
or a pharmaceutically acceptable salt thereof.

4. A pharmaceutically acceptable salt according to any one of claims 1 to 3 wherein the salt is a hydrochloride, hydrobromide, hydroiodide, phosphate, nitrate, sulfate, acetate or citrate salt.

5. A pyridine derivative according to any one of claims 1to 3 or a pharmaceutically acceptable salt according to any one of claims 1 to 4 in the form of a hydrate or a solvate.

6. A process of producing a pyridine derivative of formula (I) or a pharmaceutically acceptable salt thereof as defined in any one of claims 1 to 5 which process comprises reacting the compound of formula (II): 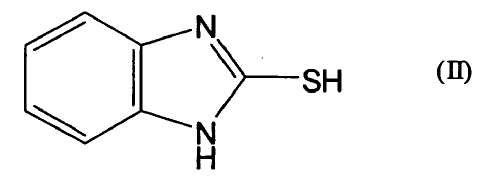 with a compound of formula (III): 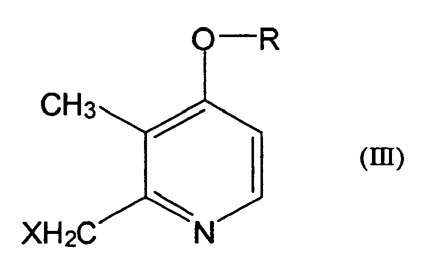 wherein R is as defined above; and X is a halogen atom or a sulfonyloxy group.

7. A pharmaceutical composition comprising a pyridine derivative or a pharmaceutically acceptable salt according to any one of claims 1 to 5 and a pharmaceutically acceptable carrier, excipient or additive.

8. A pharmaceutical composition according to claim 7 further comprising one or two or more dextrins.

9. A pharmaceutical composition according to claim 7 or claim 8, further comprising one or two or more drugs for suppressing the secretion of gastric acid.

10. A pharmaceutical composition according to claim 8 or claim 9 comprising at least one dextrin selected from α-dextrin, β-dextrin, γ-dextrin, α-cyclodextrin, β-cyclodextrin and γ-cyclodextrin and/or at least one drug for suppressing the secretion of gastric acids selected from famotidine, ranitidine, lansoprazole, omeprazole, rabeprazole and pantoprazole.

11. A derivative or a pharmaceutically acceptable salt thereof according to any one of claims 1 to 5 or a pharmaceutical composition according to any one of claims 7 to 10 for use in a method of treatment or diagnosis practised on the human or animal body.

12. A derivative or a pharmaceutically acceptable salt thereof according to any one of claims 1 to 5 or a pharmaceutical composition according to any one of claims 7 to 10 for use in propyhylactic or therapeutic treatment of a disease associated with Helicobacter pylori.

13. A derivative or a pharmaceutically acceptable salt thereof or a pharmaceutical composition for use according to claim 12, wherein the disease is associated with a Helicobacter pylori bacterium which is resistant to macrolide-based antibiotic substances or quinolone-based antibiotic substances,

14. A derivative or a pharmaceutically acceptable salt thereof or pharmaceutical composition for use according to claims 12 or 13, wherein the disease is gastritis, gastric ulcer, duodenal ulcer, non-ulcer dyspepsia syndrome, gastric MALT lymphoma, hyperplastic polyp of the stomach, gastric cancer, digestive system cancer, pancreatitis or inflammatory bowel disease.

15. A derivative or a pharmaceutically acceptable salt thereof or a pharmaceutical composition for use according to claims 12 or 13, wherein the disease is a gastric cancer developing after endoscopic excision of early gastric cancer.

## Patentansprüche

1. Pyridin-Derivat der Formel (I): 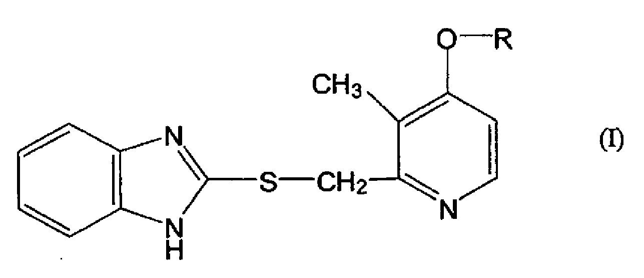 worin R eine geradkettige Hydroxylalkylgruppe mit 5 bis 10 Kohlenstoffatomen ist, oder ein pharmazeutisch akzeptables Salz davon.

2. Derivat nach Anspruch 1, worin R eine endständige Hydroxylgruppe aufweist, oder ein pharmazeutisch akzeptables Salz davon.

3. Pyridin-Derivat nach Anspruch 1 oder Anspruch 2 und ausgewählt aus
2-[{4-(5-Hydroxypentyloxy)-3-methylpyridin-2-yl}-methylthio]-1H-benzimidazol,
2-[{4-(6-Hydroxyhexyloxy)-3-methylpyridin-2-yl}-methylthio]-1H-benzimidazol,
2-[{4-(7-Hydroxyheptyloxy)-3-methylpyridin-2-yl}-methylthio]-1H-benzimidazol,
2-[{4-(8-Hydroxyoctyloxy)-3-methylpyridin-2-yl}-methylthio]-1H-benzimidazol,
2-[{4-(9-Hydroxynonyloxy)-3-methylpyridin-2-yl}-methylthio]-1H-benzimidazol,
2-[{4-(10-Hydroxydecyloxy)-3-methylpyridin-2-yl}-methylthio]-1H-benzimidazol,
oder ein pharmazeutisch akzeptables Salz davon.

4. Pharmazeutisch akzeptables Salz nach jedem der Ansprüche 1 bis 3, wobei das Salz ein Hydrochlorid-, Hydrobromid-, Hydroiodid-, Phosphat-, Nitrat-, Sulfat-, Acetat- oder Citratsalz ist.

5. Pyridin-Derivat nach jedem der Ansprüche 1 bis 3 oder ein pharmazeutisch akzeptables Salz nach jedem der Ansprüche 1 bis 4 in der Form eines Hydrats oder eines Solvats.

6. Prozess zur Herstellung eines Pyridin-Derivats der Formel (I) oder eines pharmazeutisch akzeptablen Salzes davon, wie in jedem der Ansprüche 1 bis 5 definiert, welcher Prozess das Umsetzen der Verbindung der Formel (II): 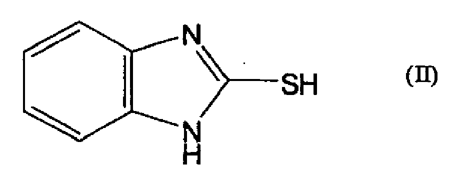 mit einer Verbindung der Formel (III): 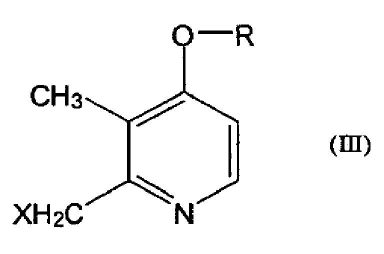 umfasst, worin R wie oben definiert ist; und X ein Halogenatom oder eine Sulfonyloxygruppe ist.

7. Pharmazeutische Zusammensetzung, umfassend ein Pyridin-Derivat oder ein pharmazeutisch akzeptables Salz nach jedem der Ansprüche 1 bis 5 und einen pharmazeutisch akzeptablen Träger, Exzipienten oder Additiv.

8. Pharmazeutische Zusammensetzung nach Anspruch 7, außerdem umfassend ein oder zwei oder mehrere Dextrine.

9. Pharmazeutische Zusammensetzung nach Anspruch 7 oder Anspruch 8, außerdem umfassend ein oder zwei oder mehrere Wirkstoffe zur Unterdrückung der Sekretion von Magensäure.

10. Pharmazeutische Zusammensetzung nach Anspruch 8 oder Anspruch 9, umfassend wenigstens ein Dextrin, ausgewählt aus α-Dextrin, β-Dextrin, γ-Dextrin, α-Cyclodextrin, β-Cyclodextrin und γ-Cyclodextrin und/oder wenigstens einen Wirkstoff zur Unterdrückung der Sekretion von Magensäuren, ausgewählt aus Famotidin, Ranitidin, Lansoprazol, Omeprazol, Rabeprazol und Pantoprazol.

11. Derivat oder ein pharmazeutisch akzeptables Salz davon nach jedem der Ansprüche 1 bis 5 oder eine pharmazeutische Zusammensetzung nach jedem der Ansprüche 7 bis 10 zur Verwendung bei einer Behandlungs- oder Diagnose-Methode, die an dem menschlichen oder tierischen Körper ausgeübt wird.

12. Derivat oder ein pharmazeutisch akzeptables Salz davon nach jedem der Ansprüche 1 bis 5 oder eine pharmazeutische Zusammensetzung nach jedem der Ansprüche 7 bis 10 zur Verwendung in der prophylaktischen oder therapeutischen Behandlung einer mit Helicobacter pylori in Verbindung stehenden Erkrankung.

13. Derivat oder ein pharmazeutisch akzeptables Salz davon oder eine pharmazeutische Zusammensetzung zur Verwendung nach Anspruch 12, wobei die Erkrankung mit einem Helicobacter pylori-Bakterium in Verbindung steht, welches resistent gegenüber Makrolid-basierten antibiotischen Substanzen oder Chinolon-basierten antibiotischen Substanzen ist.

14. Derivat oder ein pharmazeutisch akzeptables Salz davon oder eine pharmazeutische Zusammensetzung zur Verwendung nach Ansprüchen 12 oder 13, wobei die Erkrankung Gastritis, Magengeschwür, Duodenalgeschwür, Non-Ulcer-Dyspepsie-Syndrom (Reizmagen-Syndrom), gastrisches MALT-Lymphom, hyperplastischer Polyp des Magens, Magenkrebs, Krebs des Verdauungssystems, Pankreatitis oder entzündliche Darmerkrankung ist.

15. Derivat oder ein pharmazeutisch akzeptables Salz davon oder eine pharmazeutische Zusammensetzung zur Verwendung nach Ansprüchen 12 oder 13, wobei die Erkrankung ein Magenkrebs ist, der sich nach endoskopischer Exzision eines Magenkrebses im Frühstadium entwickelt.

## Revendications

1. Dérivé de pyridine de formule (I) : 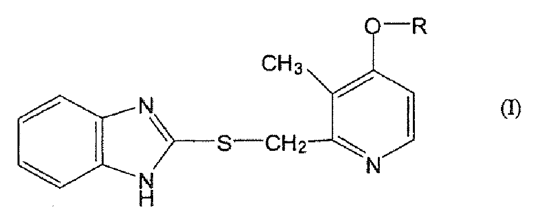 dans laquelle R est un groupe hydroxyalkyle à chaîne droite ayant de 5 à 10 atomes de carbone, ou sel pharmaceutiquement acceptable d'un tel dérivé.

2. Dérivé selon la revendication 1, dans lequel R comporte un groupe hydroxy terminal, ou sel pharmaceutiquement acceptable d'un tel dérivé.

3. Dérivé de pyridine selon la revendication 1 ou la revendication 2, et choisi parmi le 2-[{4-(5-hydroxypentyloxy)-3-méthylpyridin-2-yl}méthyl-thio]-1H-benzimidazole, le 2-[{4-(6-hydroxyhexyloxy)-3-méthylpyridin-2-yl}-méthylthio]-1H-benzimidazole, le 2-[{4-(7-hydroxyheptyloxy)-3-méthyl-pyridin-2-yllméthylthio]-1H-benzimidazole, le 2-[{4-(8-hydroxyoctyloxy)-3-méthylpyridin-2-yl}méthylthio]-1H-benzimidazole, le 2-[{4-(9-hydroxy-nonyloxy)-3-méthylpyridin-2-yl}méthylthio]-1H-benzimidazole, le 2-[{4-(10-hydroxydécyloxy)-3-méthylpyridin-2-yl}méthylthio]-1H-benzimidazole ou sel pharmaceutiquement acceptable d'un tel dérivé.

4. Sel pharmaceutiquement acceptable selon l'une quelconque des revendications 1 à 3, le sel étant un chlorhydrate, bromhydrate, iodhydrate, phosphate, nitrate, acétate ou citrate.

5. Dérivé de pyridine selon l'une quelconque des revendications 1 à 3 ou sel pharmaceutiquement acceptable selon l'une quelconque des revendications 1 à 4, sous la forme d'un hydrate ou d'un produit de solvatation.

6. Procédé de préparation d'un dérivé de pyridine de formule (I) ou d'un sel pharmaceutiquement acceptable d'un tel dérivé, tel que défini dans l'une quelconque des revendications 1 à 5, lequel procédé comprend la mise en réaction du composé de formule (II) : 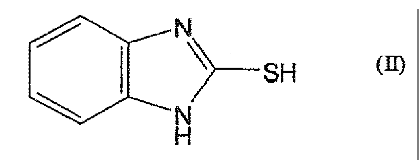 avec un composé de formule (III) : 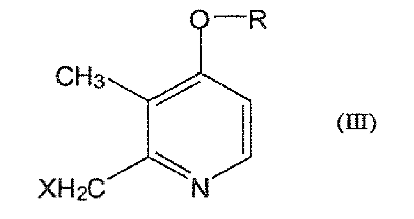 dans laquelle R est tel que défini plus haut, et X est un atome d'halogène ou un groupe sulfonyloxy.

7. Composition pharmaceutique comprenant un dérivé de pyridine ou un sel pharmaceutiquement acceptable selon l'une quelconque des revendications 1 à 5 et un véhicule, excipient ou additif pharmaceutiquement acceptable.

8. Composition pharmaceutique selon la revendication 7, comprenant en outre une ou deux ou plus de deux dextrines.

9. Composition pharmaceutique selon la revendication 7 ou la revendication 8, comprenant en outre un ou deux ou plus de deux principes actifs pour la suppression de la sécrétion d'acide gastrique.

10. Composition pharmaceutique selon la revendication 8 ou la revendication 9, comprenant au moins un dérivé de dextrine choisi parmi l'α-dextrine, la β-dextrine, la γ-dextrine, l'α-cyclodextrine, la β-cyclodextrine et la γ-cyclodextrine et/ou au moins un principe actif pour la suppression de la sécrétion d'acide gastrique, choisi parmi la famotidine, la ranitidine, le lansoprazole, l'oméprazole, le rabéprazole et le pantoprazole.

11. Dérivé ou sel pharmaceutiquement acceptable d'un tel dérivé selon l'une quelconque des revendications 1 à 5 ou composition pharmaceutique selon l'une quelconque des revendications 7 à 10, pour utilisation dans un procédé de traitement ou de diagnostic pratiqué sur le corps humain ou animal.

12. Dérivé ou sel pharmaceutiquement acceptable d'un tel dérivé selon l'une quelconque des revendications 1 à 5 ou composition pharmaceutique selon l'une quelconque des revendications 7 à 10, pour utilisation dans le traitement prophylactique ou thérapeutique d'une maladie associée à *Helicobacter pylori.*

13. Dérivé ou sel pharmaceutiquement acceptable d'un tel dérivé ou composition pharmaceutique pour utilisation selon la revendication 12, la maladie étant associée à une bactérie *Helicobacter pylori* qui est résistante à des substances antibiotiques à base de macrolides ou des substances antibiotiques à base de quinolone.

14. Dérivé ou sel pharmaceutiquement acceptable d'un tel dérivé ou composition pharmaceutique pour utilisation selon la revendication 12 ou 13, la maladie étant la gastrite, l'ulcère gastrique, l'ulcère duodénal, le syndrome de dyspepsie non ulcéreuse, le lymphome du MALT gastrique, le polype hyperplasique de l'estomac, le cancer gastrique, le cancer du système digestif, la pancréatite ou la maladie intestinale inflammatoire.

15. Dérivé ou sel pharmaceutiquement acceptable d'un tel dérivé ou composition pharmaceutique pour utilisation selon la revendication 12 ou 13, la maladie étant un cancer gastrique se développant après excision endoscopique d'un cancer gastrique antérieur.
